(19) Europäisches Patentamt
European Patent Office
Office européen des brevets

(11) EP 2 737 065 B1

(12) **EUROPEAN PATENT SPECIFICATION**

(45) Date of publication and mention of the grant of the patent:
**25.04.2018 Bulletin 2018/17**

(21) Application number: **12819628.4**

(22) Date of filing: **27.07.2012**

(51) Int Cl.:
***C12N 15/63*** *(2006.01)* ***C07K 19/00*** *(2006.01)*
***C07K 14/195*** *(2006.01)* ***C07K 1/34*** *(2006.01)*
***C07K 1/18*** *(2006.01)* ***C07K 1/36*** *(2006.01)*

(86) International application number:
**PCT/US2012/048498**

(87) International publication number:
**WO 2013/019604 (07.02.2013 Gazette 2013/06)**

(54) **PROTEIN PRODUCTION METHOD**

PROTEINHERSTELLUNGSVERFAHREN

PROCÉDÉ DE PRODUCTION DE PROTÉINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2011 US 201161513281 P**

(43) Date of publication of application:
**04.06.2014 Bulletin 2014/23**

(73) Proprietor: **Georgia State University Research Foundation, Inc.**
**Atlanta, GA 30303 (US)**

(72) Inventors:
- **PIERCE, George, F.**
  **Canton, Georgia 30115 (US)**
- **BURRAN, Susan, A.**
  **Decatur, Georgia 30030 (US)**
- **TUCKER, Trudy, Ann**
  **Atlanta, Georgia 30306 (US)**
- **JONES-DOZIER, Shelby**
  **Mableton, Georgia 30126 (US)**
- **HOOKER, Jennifer**
  **Acworth, Georgia 30102 (US)**
- **CROW, Sidney, A., Jr.**
  **Smyrna, Georgia 30080 (US)**

(74) Representative: **Lavoix**
**Bayerstrasse 83**
**80335 München (DE)**

(56) References cited:
**WO-A2-03/080660 US-A- 5 162 507**
**US-A- 5 437 986 US-A1- 2003 166 062**

- **SINGH S M ET AL: "Solubilization and refolding of bacterial inclusion body proteins", JOURNAL OF BIOSCIENCE AND BIOENGINEERING, ELSEVIER, AMSTERDAM, NL, vol. 99, no. 4, 1 April 2005 (2005-04-01), pages 303-310, XP027707167, ISSN: 1389-1723 [retrieved on 2005-04-01]**
- **RUDOLPH R ET AL: "IN VITRO FOLDING OF INCLUSION BODY PROTEINS", FASEB JOURNAL, FED. OF AMERICAN SOC. FOR EXPERIMENTAL BIOLOGY, US, vol. 10, no. 1, 1 January 1996 (1996-01-01), pages 49-56, XP000605154, ISSN: 0892-6638**
- **CLARK E D B: "Protein refolding for industrial processes", CURRENT OPINION IN BIOTECHNOLOGY, LONDON, GB, vol. 12, no. 2, 1 April 2001 (2001-04-01), pages 202-207, XP002485975, ISSN: 0958-1669, DOI: 10.1016/S0958-1669(00)00200-7**
- **CABRITA L D ET AL: "PROTEIN EXPRESSION AND REFOLDING - A PRACTICAL GUIDE TO GETTING THE MOST OUT OF INCLUSION BODIES", BIOTECHNOLOGY ANNUAL REVIEW, ELSEVIER, NL, vol. 10, no. SPEC. ISSUE, 1 January 2004 (2004-01-01), pages 31-50, XP009045245, ISSN: 1387-2656, DOI: 10.1016/S1387-2656(04)10002-1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 737 065 B1

## Description

## BACKGROUND

Recombinant proteins have been produced for over thirty years. Generally, the goal has been to produce soluble recombinant protein, while largely ignoring insoluble protein produced in host cells. Insoluble proteins are viewed as being protein inclusion bodies without biological activity or as difficult to renature. Therefore, most efforts have been focused on maximizing the amount of soluble protein, rather than developing methods to enhance the amount of functional recombinant protein obtained from insoluble protein or inclusion bodies in host cells.

## SUMMARY

Provided herein are methods of producing a heterologous polypeptide and compositions comprising same. More specifically, provided herein is a method of producing a heterologous polypeptide, the method comprising the steps of: a) growing Gram-negative bacteria comprising a nucleotide sequence encoding a heterologous polypeptide operably linked to an inducible promoter under fed-batch fermentation conditions in a synthetic medium; b) inducing expression of the heterologous polypeptide; c)harvesting the bacteria in the synthetic medium by decanting; d) homogenizing the bacteria contained in the synthetic medium; e) obtaining from the synthetic medium comprising the homogenized bacteria of step d) a soluble fraction comprising the heterologous polypeptide and an insoluble fraction comprising the heterologous polypeptide; f) resuspending the insoluble fraction; g) centrifuging the resuspended insoluble fraction of step g) to obtain a supernatant comprising the heterologous polypeptide; h) denaturing the polypeptide in the supernatant obtained from step g); i) refolding the denatured polypeptide of step h); and j) subjecting the refolded polypeptide to anion exchange chromatography to obtain the heterologous polypeptide.

The invention to which the present specification pertains is set out in the claims appended to this description.

## DETAILED DESCRIPTION

Provided herein is a method of producing a heterologous polypeptide, the method comprising the steps of: a) growing Gram-negative bacteria comprising a nucleotide sequence encoding a heterologous polypeptide operably linked to an inducible promoter under fed-batch fermentation conditions in a synthetic medium; b) inducing expression of the heterologous polypeptide; c) harvesting the bacteria in the synthetic medium by decanting; d) homogenizing the bacteria contained in the synthetic medium; e) obtaining from the synthetic medium comprising the homogenized bacteria of step d) a soluble fraction comprising the heterologous polypeptide and an insoluble fraction comprising the heterologous polypeptide; f) resuspending the insoluble fraction; g) centrifuging the resuspended insoluble fraction of step g) to obtain a supernatant comprising the heterologous polypeptide; h) denaturing the polypeptide in the supernatant obtained from step g); i) refolding the denatured polypeptide of step h); and j) subjecting the refolded polypeptide to anion exchange chromatography to obtain the heterologous polypeptide.

The method can further comprise k) precipitating the heterologous polypeptide from the soluble fraction of step e) in a precipitate; l) resuspending the polypeptide precipitate from step k); m) centrifuging the resuspended polypeptide precipitate to obtain a supernatant comprising the heterologous polypeptide; n) subjecting the supernatant obtained in step m) to tangential flow filtration to form a filtration product; o) performing a two-phase separation on the filtration product of step n) to produce a an aqueous phase and a detergent phase; p) denaturing the polypeptide in the aqueous phase obtained in step o); q) refolding the denatured polypeptide of step p); and r) subjecting the refolded polypeptide to anion exchange chromatography to obtain the heterologous polypeptide.

As used throughout, a heterologous polypeptide is a polypeptide that is not normally expressed in a host cell. A heterologous polypeptide can also be a recombinant protein produced by a recombinant nucleic acid that is artificially introduced into a host cell. The host cell can be a prokaryotic cell or a eukaryotic cell. For example, a heterologous polypeptide can be a polypeptide that is derived from an infectious agent, another cell type or organism and is not normally expressed in Gram-negative bacteria. Expression of the heterologous polypeptide is achieved by introduction or transformation of the nucleotide sequence encoding the heterologous polypeptide into a Gram-negative bacterial host via standard techniques. The heterologous polypeptide is not limited to any specific type of heterologous polypeptide as a wide variety of heterologous polypeptides can be produced by the methods disclosed herein including, for example, heterologous polypeptides comprising proteins or protein fragments useful for human or veterinary therapy as well as diagnostic applications. These include, but are not limited to, viral proteins, bacterial proteins, parasitic proteins, fungal proteins, hormones, growth or inhibitory factors, enzymes or fragments thereof. The heterologous polypeptide can comprise one or more antigenic proteins or antigenic protein fragments.

The heterologous polypeptide can comprise any viral protein or fragment thereof derived from an RNA virus (including negative stranded RNA viruses, positive stranded RNA viruses, double stranded RNA viruses and retroviruses) or a DNA virus. Proteins from all strains, types, subtypes of DNA and RNA viruses are contemplated herein. Examples of RNA viruses include influenza viruses such as influenza A, influenza B, or influenza C viruses. Influenza proteins or fragments thereof from H1N1, H2N2, H3N2, H5N1, H7N7, H1N2, H9N2, H7N2, H7N3 and H10N7 are also contemplated herein. Proteins or fragments thereof from avian influenza strains such as H5N1, H5N1 Duck/MN/1525/81, H5N2, H7N1, H7N7 and H9N2 are also provided. Hemagglutinin (HA), neuraminidase (NA) proteins and fragments thereof from influenza viruses are also provided.

Other examples of RNA viruses include, but are not limited to, picornaviruses, enteroviruses (for example polio viruses 1, 2 and 3), rhinovirus (for example, rhinovirus A, rhinovirus B, rhinovirus C, human rhinovirus 1-100 and bovine rhinoviruses 1-3). Other examples include noroviruses (for example, Norwalk virus), alphaviruses (for example, Chikungunya virus, Sindbis virus, Semliki Forest virus), rubella viruses, flaviviruses, (for example, Dengue virus (types 1 to 4), Japanese encephalitis virus (JEV), West Nile virus (WNV), Yellow fever virus, hepacivirus (hepatitis C virus (HCV)) all six genotypes) and bovine viral diarrhea virus (BVDV) types 1 and 2.). Other examples include coronaviruses (for example, SARS-CoV), filoviruses (for example Marburg and Ebola viruses such as, EBOV-Z, EBOV-S, EBOV-IC and EBOV-R), mumps, Newcastle disease virus, pneumoviruses (for example, human respiratory syncytial virus A2, B1 and S2 and bovine respiratory syncytial virus), othromyxoviruses, reoviruses, rotaviruses, retroviruses, lentiviruses (for example, human immunodeficiency virus (HIV) type 1, human immunodeficiency virus (HIV) type 2, human immunodeficiency virus (HIV) type 3, simian immunodeficiency virus, equine infectious anemia virus and feline immunodeficiency virus) and spumaviruses (for example, human foamy virus and feline syncytia- forming virus).

Examples of DNA viruses include polyomaviruses, papillomaviruses (for example, human papillomavirus, or bovine papillomavirus), adenoviruses (for example, adenoviruses A-F), circoviruses (for example, porcine circovirus and beak and feather disease virus (BFDV)), parvoviruses (for example, canine parvovirus), erythroviruses (for example, adeno-associated virus types 1-8), herpes viruses 1,2, 3, 4, 5, 6, 7 and 8 (for example, herpes simplex virus 1, herpes simplex virus 2, varicella-zoster virus, Epstein-Barr virus, cytomegalovirus, Kaposi's sarcoma associated herpes virus, and herpes virus 1 (B virus)), poxviruses (for example, smallpox (variola), cowpox, monkeypox, vaccinia, and swinepox), and hepadnaviruses (for example, hepatitis B and hepatitis B-like viruses).

The heterologous polypeptide can comprise any bacterial protein or fragment thereof. For example, and not to be limiting, the bacterial protein or fragment thereof can be derived from the following bacteria: *Listeria* (*sp.*), *Franscicella tularensis, Mycobacterium tuberculosis, Rickettsia* (all types), *Ehrlichia, Chylamida.* Other examples include *M. tuberculosis, M. bovis, M. avium, M. intracellulare, M. africanum, M. kansasii, M. marinum, M. ulcerans, M. avium* subspecies *paratuberculosis, Nocardia asteroides,* other *Nocardia* species, *Legionella pneumophila,* other *Legionella* species, *Salmonella typhi,* other *Salmonella* species, *Shigella* species, *Yersinia pestis, Pasteurella haemolytica, Pasteurella multocida,* other *Pasteurella species, Actinobacillus pleuropneumoniae, Listeria monocytogenes, Listeria ivanovii, Brucella abortus,* other *Brucella* species, *Cowdria ruminantium, Chlamydia pneumoniae, Chlamydia trachomatis, Chlamydia psittaci, Coxiella burnetti,* other *Rickettsial* species, *Ehrlichia* species, *Staphylococcus aureus* (for example, MRSA), *Staphylococcus epidermidis, Streptococcus pyogenes, Streptococcus agalactiae, Bacillus anthracis, Escherichia coli, Vibrio cholerae, Kingella kingae, Campylobacter* species, *Neiserria meningitidis, Neiserria gonorrhea, Pseudomonas aeruginosa,* other *Pseudomonas* species, *Haemophilus influenzae, Haemophilus ducreyi,* other *Hemophilus* species, *Clostridium tetani,* other *Clostridium* species, *Yersinia enterolitica,* and other *Yersinia* species.

The heterologous polypeptide can comprise any parasitic protein or fragment thereof. For example, and not to be limiting, the parasitic protein or fragment thereof can be derived from the following parasites: *Cryptosporidium, Plasmodium* (all species), American trypanosomes (*T. cruzi*), African trypanosomes, *Acanthamoeba, Entaoeba histolytica, Angiostrongylus, Anisakis, Ascaris, Babesia, Balantidium, Baylisascaris, Capillaria, Clonorchis, Chilomastix mesnili, Cyclspora, Diphyllobothrium, Dipylidium caninum, Fasciola, Giardia, Gnathostoma, Hetetophyes, Hymenolepsis, Isospora, Loa loa, Microsporidia, Naegleria, Toxocara, Onchocerca, Opisthorchis, Paragonimus, Baylisascaris, Strongyloides, Taenia, Trichomonas and Trichuris.*

The heterologous polypeptide can comprise a protozoal or fungal protein or a fragment thereof. For example, the protein can be derived from *Plasmodium falciparum,* other *Plasmodium* species, *Toxoplasma gondii, Pneumocystis carinii, Trypanosoma cruzi,* other trypanosomal species, *Leishmania donovani,* other *Leishmania species, Theileria annulata,* other *Theileria* species, *Eimeria tenella,* other *Eimeria* species, *Histoplasma capsulatum, Cryptococcus neoformans, Blastomyces dermatitidis*, *Coccidioides immitis, Paracoccidioides brasiliensis, Penicillium marneffei,* and *Candida* species.

The heterologous polypeptide can also be a fusion protein, for example, a fusion protein comprising a flagellin or a fragment thereof, and at least one antigenic peptide that elicits an immune response, including eliciting an antibody response. The antigenic peptide can be a full-length protein or a fragment thereof. The antigenic peptide can be of any length. For example, the peptide can comprise from about 10 amino acids to about 25 amino acids, from about 25 amino acids to about 50 amino acids, from about 50 amino acids to about 100 amino acids, from about 100 amino acids to about 200 amino acids, or from about 200 amino acids to about 400 amino acids. The antigenic peptide of the fusion proteins described herein can be an antigenic peptide that elicit antibodies against a bacteria, a virus, a parasite or a fungus upon administration to an organism, for example a mammal.

The flagellin or flagellin fragment can be any flagellin or flagellin fragment that is recognized by Toll Like Receptor 5 (TLR5). The flagellin can be a FliC flagellin, a FlaC flagellin, a FljB flagellin or a fragment thereof. For example, a flagellin or flagellin fragment derived from Gammaproteobacteria can be utilized. These include, but are not limited to flagellins from *Salmonella, E. coli, Yersinia, Vibrio, Pseudomonas aeruginosa* and *Klebsiella pneumoniae.* In the fusion proteins described herein, the peptide can be linked to the N-terminus of a flagellin or a fragment thereof or linked to the C-terminus of a flagellin or a fragment thereof. A fusion protein can also comprise more than one flagellin or a fragment thereof. For example, the fusion protein can comprise a peptide, wherein the N-terminus and the C-terminus of the peptide are linked to a flagellin or a fragment thereof. The fusion protein can further comprise linker sequences that link one or more flagellins or fragment thereof to one or more antigenic peptides. The linker sequences can vary in length, and can be, for example, from 1 amino acid to 100 amino acids in length, or greater. Appropriate linker sequences for fusion proteins can be determined by one of skill in the art, for example by utilizing LINKER (See Crasto and Feng, "LINKER: a program to generate linker sequences for fusion proteins," PEDS, 13(5): 309-312 (2000)).

An example of a FliC flagellin polypeptide sequence from *Salmonella typhimurium* LT2 is set forth herein as SEQ ID NO: 1. The nucleotide sequence encoding SEQ ID NO: 1 is set forth herein as SEQ ID NO: 2. Fusion proteins comprising SEQ ID NO: 1 or a fragment thereof and at least one antigenic peptide are provided herein. For example, a fusion protein can comprise SEQ ID NO: 1 or a fragment thereof and an antigenic epitope from any virus, bacteria, parasite or fungus. Fragments of SEQ ID NO: 1, can comprise, for example, amino acids 1-100, 1-200, 1-300, 1-400, 1-425, 1-450, 1-455, 1-460, 1-465, 1-470 or 1-475 of SEQ ID NO: 1. For example, a fusion protein comprising SEQ ID NO:1 or a fragment thereof, and an antigenic influenza epitope is disclosed. An antigenic influenza epitope can be an HA protein or an HA protein fragment. For example, the antigenic influenza epitope can be from the globular head region of an HA protein, or a fragment thereof (See Russell et al. "The global circulation of seasonal influenza A (H3N2) viruses," Science 320(5874): 340-346 (2008); and Chaipan et al. "Proteolytic activation of the 1918 Influenza Virus Hemagglutinin Journal of Virology, 83(7): 3200-3211 (2009). An HA protein sequence is provided herein as SEQ ID NO: 6. Antigenic fragments of SEQ ID NO: 6 can also be utilized. The nucleotide sequence encoding SEQ ID NO: 6 is provided herein as SEQ ID NO: 7. In another example, a fusion protein can also comprise SEQ ID NO: 1 or a fragment thereof and an antigenic Marburg epitope. An example is provided herein as SEQ ID NO: 9. An antigenic Marburg epitope can be, for example, a polypeptide comprising the amino acid sequence set forth as I L I Q G T K N L P I L E I A(SEQ ID NO: 3). a polypeptide comprising the amino acid sequence set forth as E P H Y S P L I L A L K T L E (SEQ ID NO: 4) or a polypeptide comprising the amino acid sequence set forth as G I L L L L S I A V L I A L S (SEQ ID NO: 5). See Kalina et al., "Discovery of common marburgvirus protective epitopes in BALB/c mouse model," Virology Journal 6:132 (2009) for examples of Marburg epitopes.

Numerous Gram-negative bacteria can be utilized in the methods set forth herein. These include, but are not limited to, *E. coli, Agrobacterium, Salmonella* (for example, *Salmonella typhimurium*) *Pseudomonas* (for example, *Pseudomonas fluorescens and Pseudomonas aeruginosa*) and *Bacillus* (for example, *Bacillus spp.*) strains. Examples of *E. coli* strains include BL21(DE3), BL21(DE3)-pLysS, BL21 Star-pLysS, BL21-SI, BL21-AI, BL21 codon plus, Tuner, Tuner pLysS, Rosetta, Rosetta pLysS, Rosetta-gami-pLysS, Origami, Origami B, Origami B pLysS, AD494, BL21trxB, HMS174, Novablue(DE3), BLR, C41(DE3), and C43(DE3).

The host cells, for example, Gram-negative bacteria, utilized in the methods set forth herein comprise a nucleotide sequence encoding a heterologous polypeptide. The nucleotide sequence encoding a heterologous polypeptide can be in a vector. The vector is contemplated to have the necessary functional elements that direct and regulate transcription of the inserted nucleic acid. These functional elements include, but are not limited to, a promoter, a repressor region, regions upstream or downstream of the promoter, such as enhancers that can regulate the transcriptional activity of the promoter, an origin of replication, appropriate restriction sites to facilitate cloning of inserts adjacent to the promoter, antibiotic resistance genes or other markers which can serve to select for cells containing the vector or the vector containing the insert, RNA splice junctions, a transcription termination region, or any other region which can serve to facilitate the expression of the inserted nucleotide sequence The vector, for example, can be a plasmid. The vectors can contain genes conferring hygromycin resistance, ampicillin resistance, gentamicin resistance, neomycin resistance or other genes or phenotypes suitable for use as selectable markers.

There are numerous other *E. coli* (*Escherichia coli*) expression vectors, known to one of ordinary skill in the art, which are useful for the expression of the nucleic acid insert. For example, vectors comprising a T7 promoter system, a lactose promoter system (lac), a tryptophan (Trp) promoter system, a hybrid trp-lac promoter system, a beta-lactamase promoter system, or a phage lambda promoter system can be utilized. Inducible promoter systems can also be employed. For example, and not to be limiting, an IPTG-inducible promoter, a sugar-inducible promoter, a temperature-inducible promoter or a copper inducible promoter can be used. In addition to expression in Gram-negative bacterial cells, the nucleic acid encoding a heterologous polypeptide can be expressed in gram positive bacterial cells or eukaryotic cells, for example, yeast cells. More specifically, the nucleic acid can be expressed by *Pichia pastoris* or *S. cerevisiae.*

The Gram-negative bacteria can be grown or cultured under a batch fermentation process, a fed-batch fermentation process, or a combination thereof. The growth time for the fermentation process will vary, but can be from about 12 to 48 hours, for the combination of a batch fermentation process and a fed batch fermentation process. This includes, but is not limited to growth times of about 12-24 hours, 12 to 36 hours, 12 to 48 hours, 18 to 24 hours, 18 to 36 hours and 18 to 48 hours. The growth time for the fed batch fermentation process will vary, but can be from about 1 to 8 hours, from about 1 to 6 hours, from about 1 to 4 hours, or from about 1 to 2 hours. The Gram-negative bacteria can be grown under batch fermentation conditions followed by growth under fed batch fermentation conditions. In batch fermentation, growth medium is inoculated with bacteria and no additional substrate is added throughout the fermentation process. Fed batch fermentation involves controlled feeding of one or more growth limiting nutrients to a culture. The controlled, or incremental addition of the growth limiting nutrient(s) affects the growth rate of the culture and allows metabolic control to avoid excess formation of acetate, lactic acid and other metabolites in the culture. For example, a carbon source (glucose or glycerol) can be metered in, in a limited manner such that the formation of growth-inhibiting by-products, for example acetate, is avoided, with the consequence that the growth can be continued in a manner which is only substrate-limited until high cell densities are reached.

As used herein, the term "culture" refers to at least one medium containing at least one host cell, for example, a Gram-negative bacterial strain, which is suitable for fermentation. It is to be understood that the term "culture" as used herein includes a medium wherein all necessary components are added to the medium before the start of the fermentation process, and further includes a medium wherein part of the necessary components are added before starting and part are added to the medium during the fermentation process. As used herein, the term "medium" refers to a nutrient system for the cultivation of cells. Nutrients that can be fed to a fermentation process include carbon/energy sources, such as glucose, carbohydrates, vitamins, minerals, oils, and the like. The medium can be a defined medium, also known as a synthetic medium, wherein all of the chemicals used are known and there is no yeast, animal or plant tissue present. Examples of synthetic media that can be utilized in the methods described herein are provided in the Examples section below. It is also contemplated that, prior to growing the bacteria in a synthetic medium, the Gram-negative bacteria can be grown in undefined medium comprising complex ingredients, such as yeast extract or casein hydrolysate, which contain a mixture of chemical species in unknown proportions.

Any fermentation vessel suitable for propagation of bacteria can be utilized. For example, a conical fermenter, a closed-top fermentation tank or an open-top fermentation tank can be used. The fermenter can be equipped with or attached to a measuring device, a display device, a computer control device and/or a metering device. For example, numerous parameters, such as acid/base concentration, foam, air-air/oxygen mix, agitation, dissolved oxygen and temperature can be monitored and controlled.

During fermentation, the pH of the fermentation medium is maintained, to between about 6.5 and 7.2, or between about 6.5 and 7.0. Therefore, the pH can be about 6.5, 6.6, 6.7, 6.8, 6.9, 7.0, 7.1 or 7.2. For example, the pH can be about 7.0. The concentration of the carbon source during the batch phase and/or the fed-batch phase can be in a range from about 0.01 to about 35g/l. For example, the concentration can range from about 0.5 to 25 g/l, from about 1 g/l to 20 g/l, from about 1.0 g/l to 15 g/l, from about 1.0 g/l to 5 g/l, from about 1.0 to 3 g/l, from about 5g/l to 25g/l, from about 1g/l to 30g/l, from about 1g/l to 30g/l or from about 5g/l to 35g/l. For example, during the batch phase, the concentration of the carbon source, for example glucose, can be from about 5g/l to about 35g/l.

During fed batch fermentation, the carbon source can be added in a continuous manner (on-line) using an automated or semi-automated addition and analysis system. For example, for fermentation of 10 to 20 liters, a carbon source such as glucose (180mg/ml) can be added at a feed rate of 7-20ml per minute. An on-line flow injection analysis system can also be employed. In the methods set forth herein, during fed batch fermentation, glucose concentration can be maintained from about 0-0.5g/l. During fermentation, dissolved oxygen can be maintained at about 20% to about 30%. The fermentation temperature can range from about 25°C to about 37°C. For example, the temperature can be from about 25°C to about 30°C, from about 25°C to about 35°C or from about 28°C to about 30°C. In another example, the temperature can be about 30°C.

In the methods set forth herein, it is not necessary to control the rate of fermentation via a critical nutrient or a single nutrient, for example, carbon. Instead, the addition of a complete nutrient mixture in conjunction with appropriate levels of oxygen permits the culture to grow unabated at an optimum rate for an extended period of time. All nutrients can be added at the same time, so that there is no critical component limiting growth. This can be achieved by adding all or some of the nutrients together in combination or from different containers as long as all nutrients are added at the same time, so that there is no single limiting nutrient.

In the methods set forth herein, expression of the heterologous polypeptide is induced by activating the inducible promoter system, when the $OD_{600}$ of the culture is about 0.6-1.2. Alternatively, for example, in a fermenter, expression can be induced when the $OD_{600}$ of the culture is about 40 to 50. Expression can be induced when the cell density is from about 10 to 100g/l. For example, IPTG can be added to the culture to induce expression of a heterologous polypeptide under the control of an inducible lac promoter. IPTG can be added to the culture about 20 to 30 minutes after maximum feed rate is reached. The concentration of IPTG added to the culture will vary depending on the working volume of the fermenter, but the final concentration of IPTG in the culture can range from about 0.5mM to about 5mM, with a range of 1.0mM to 1.5mM preferred. In the methods disclosed herein, the majority of the recombinant proteins are expressed into the cytoplasm of the cell.

After induction, for example, about 1 to 4 hours after induction, the cells can be harvested. The cells can also be harvested when feed media is depleted. Once fed batch fermentation is complete, cell densities can range from about 50 g/l to about 150 g/l. Once fed batch fermentation is complete, the $OD_{600}$ of the culture can range from about 40 to 80. For example, the cells can be harvested when the $OD_{600}$ is about 40 to 70, about 50 to 70, or about 60 to 70.

The cells can be harvested via standard means, for example, by filtration, centrifugation or decanting. In the methods set forth herein, decanting can result in increased dry weight recovered per unit volume, which translates to decreased water. This improved dewatering results in improved cell disruption and clarification. After harvesting, the bacterial cells are homogenized by art recognized methods. Cells can be lysed by mechanical methods, for example, by utilizing a Waring blender or a Polytron or by employing pressure (*e.g.* at 10,000 psi) such as in a French Pressure Cell or with an APV cell disruptor. Cells can also be lysed by liquid homogenization or sonication. Freeze/thaw cycles can be utilized to disrupt cells through ice crystal formation. Manual grinding can also be employed. Combinations of these methods can also be used.

After homogenization, the synthetic medium comprising the homogenized bacteria can be clarified, for example by centrifugation, to obtain a soluble fraction comprising the heterologous polypeptide and an insoluble fraction comprising the heterologous polypeptide. Protocols for obtaining a soluble fraction and an insoluble fraction comprising the heterologous polypeptide are set forth in the Examples. These protocols are by no means limiting and are merely representative of how one of skill in the art can clarify the homogenate to obtain a soluble and insoluble fraction comprising the heterologous polypeptide.

Following clarification, the insoluble fraction is resuspended and centrifuged to obtain a supernatant comprising the heterologous polypeptide. This supernatant comprises the heterologous polypeptide in its native form. In its native form, the protein is in a properly folded and/or assembled form, which is operative and functional. The native form of a protein can possess all four levels of biomolecular structure (i.e., primary, secondary, tertiary and quarternary structure). This is in contrast to the denatured state, in which the secondary through quaternary structures are lost and the protein retains only the primary structure.

The polypeptide in the supernatant is denatured and then refolded. The polypeptide can be denatured by utilizing standard denaturants such as urea, guanidinium hydrochloride, SDS or cetyltrimethylammonium chloride. For example, about 4M to 8M urea can be used to denature the polypeptide. Alternatively, about 0.5 to 6.0M guanidine-HCl can be utilized.

One of skill in the art can also utilize the Novagen® Protein Refolding Kit (Catalogue No. 70123-3) to solubilize heterologous polypeptides in insoluble fractions or inclusion bodies. For example, utilizing this kit, the insoluble fraction is solubilized under denaturing (for example, in the presence of urea) or non-denaturing conditions in an N-laurylsarcosine containing buffer. Following clarification, the solubilized fraction is dialyzed against a neutral pH buffer containing a reducing agent, for example, DTT, to encourage correct disulfide bond formation. Optionally, to promote disulfide formation, one of skill in the art can perform an additional dialysis step in the presence of a redox pair, for example, oxidized and reduced glutathione or cystamine/cysteamine. A final dialysis step then removes excess reducing agent and transfers the heterologous polypeptide into the buffer of choice. It is understood that solubilization can be effected with other detergents, for example, SDS, CTAC, or CTAB in the presence or absence of a denaturing agent such as guanidinium hydrochloride or urea.

After refolding of the polypeptide, anion exchange chromatography can be used to remove any remaining bacterial cell protein/endotoxin, misfolded proteins and buffer components. Anion exchange chromatography techniques are known to those of skill in the art. Anion exchange chromatography can be performed under denaturing or non-denaturing conditions. Anion exchange chromatography can also be performed under denaturing conditions followed by refolding during purification. An example of an anion exchange protocol that can be used is set forth in the Examples. The methods set forth herein can further comprise subjecting the heterologous polypeptide to hydrophobic interaction (HIC) chromatography See, for example, Cao et al. "Purification of clinical-grade recombinant HSP65-MUCI fusion protein," Biotechnol. Appl. Biochem. 57:9-15 (2010); or Bhuvanesh et al. "Production and single-step purification of Brugia malayi abundant larval transcript (ALT-2) using hybdrophobic interaction chromatography" J. Ind. Microbl. Biotechnol. 37(10): 1053-9 (2010).

As mentioned above, a soluble fraction comprising the heterologous polypeptide and an insoluble fraction comprising the heterologous polypeptide can be obtained from homogenized bacteria. Once a soluble fraction is obtained, it can be further processed to obtain a heterologous polypeptide. The heterologous polypeptide from the soluble fraction can be precipitated, for example, by addition of polyethylene glycol (PEG) or salt precipitation (for example, ammonium sulfate precipitation). For proteins that have been salted in, desalting dialysis can be employed. The polypeptide precipitate is then resuspended and the resuspended polypeptide precipitate is centrifuged to obtain a supernatant comprising the heterologous polypeptide. The supernatant is then subjected to tangential flow filtration to form a filtration product. This is followed by a two-phase separation on the filtration product to produce an aqueous phase and a detergent phase. In this two-phase separation, Triton X-114 and PEG 3350 can be added to create an aqueous phase and a detergent phase. Triton X-114 and PEG 3350 are merely exemplary, as other detergents and polyether compounds are known to those of skill in the art. The polypeptide transitions into the aqueous phase during centrifugation. Contaminants, such as endotoxin, transition into the detergent phase. At the end of centrifugation, there is a physical separation between the aqueous peptide phase and the detergent phase. At this point, the aqueous phase is collected, for example, via decanting, and the detergent phase is discarded. The polypeptide in the aqueous phase is then denatured and refolded. The refolded polypeptide is then subjected to anion exchange chromatography to obtain the heterologous polypeptide. The aqueous phase can be filtered prior to anion exchange chromatography. It is noted that the tangential flow filtration step can be omitted and the supernatant obtained after centrifugation of the resuspended polypeptide precipitate can be directly subjected to two-phase separation.

Purification of the heterologous polypeptide fraction from the soluble fraction can further comprise subjecting the heterologous polypeptide to hydrophobic interaction (HIC) chromatography See, for example, Cao et al. "Purification of clinical-grade recombinant HSP65-MUCI fusion protein," Biotechnol. Appl. Biochem. 57():9-15 (2010); or Bhuvanesh et al. "Production and single-step purification of Brugia malayi abundant larval transcript (ALT-2) using hybdrophobic interaction chromatography" J. Ind. Microbl. Biotechnol. 37(10): 1053-9 (2010).

The heterologous polypeptides obtained by the methods disclosed herein are purified or separated from other components that naturally accompany it. Typically, it is at least 50%, by weight, free from cells, host cell proteins, organic molecules and endotoxins with which it can be associated during the fermentation process. The compound can be at least 75%, at least 90%, or at least 99%, by weight, pure.

The heterologous polypeptide obtained by the methods set forth herein can be formulated as a pharmaceutical composition, for example, as a vaccine that will elicit an immune response upon administration to a subject. The immune response can include production of antibodies, including antibodies against the heterologous polypeptide. The vaccine compositions may include a pharmaceutically acceptable carrier. By pharmaceutically acceptable is meant a material that is not biologically or otherwise undesirable, which can be administered to an individual along with the selected agent without causing unacceptable biological effects or interacting in a deleterious manner with the other components of the pharmaceutical composition in which it is contained.

As used herein, the term carrier encompasses any excipient, diluent, filler, salt, buffer, stabilizer, solubilizer, lipid, stabilizer, or other material well known in the art for use in pharmaceutical formulations. The choice of a carrier for use in a composition will depend upon the intended route of administration for the composition. The preparation of pharmaceutically acceptable carriers and formulations containing these materials is described in, e.g., Remington's Pharmaceutical Sciences, 21st Edition, ed. University of the Sciences in Philadelphia, Lippincott, Williams & Wilkins, Philadelphia Pa., 2005. Examples of physiologically acceptable carriers include buffers such as phosphate buffers, citrate buffer, and buffers with other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN® (ICI, Inc.; Bridgewater, New Jersey), polyethylene glycol (PEG), and PLURONICS™ (BASF; Florham Park, NJ).

Compositions containing the heterologous polypeptide suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. The compositions can also include adjuvants that enhance the immune response to a vaccine. Prevention of the action of microorganisms can be promoted by various antibacterial and antifungal agents, for example, parabens, chlorobutanol, phenol, sorbic acid, and the like. Isotonic agents, for example, sugars, sodium chloride, and the like may also be included. Prolonged absorption of the injectable pharmaceutical form can be brought about by the use of agents delaying absorption, for example, aluminum monostearate and gelatin.

The composition can be a liquid solution, suspension, emulsion, tablet, pill, capsule, sustained release formulation, or powder. For solid compositions (for example powder, pill, tablet, or capsule forms), conventional non-toxic solid carriers can include, for example, pharmaceutical grades of mannitol, lactose, starch, sodium saccharine, cellulose, magnesium carbonate, or magnesium stearate. The composition can be formulated as a suppository, with traditional binders and carriers such as triglycerides.

Modes of administration include but are not limited to, parenteral, mucosal, topical, intradermal, intrathecal, intratracheal, via nebulizer, via inhalation, intramuscular, intraperitoneal, vaginal, rectal, intravenous, subcutaneous, intranasal, and oral routes. Administration can be systemic or local. Pharmaceutical compositions can be delivered locally to the area in need of treatment, for example by topical application or local injection.

The disclosure also provides a pharmaceutical pack or kit comprising one or more containers filled with one or more of the ingredients of the pharmaceutical compositions. Instructions for use of the composition can also be included.

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference of each various individual and collective combinations and permutations of these compounds may not be explicitly disclosed, each is specifically contemplated and described herein. For example, if a method is disclosed and discussed and a number of modifications that can be made to the method are discussed, each and every combination and permutation of the method, and the modifications that are possible are specifically contemplated unless specifically indicated to the contrary. Thus, if there are a variety of additional steps that can be performed, it is understood that each of these additional steps can be performed with any specific method steps or combination of method steps of the disclosed methods, and that each such combination or subset of combinations is specifically contemplated and should be considered disclosed.

Ranges may be expressed herein as from about one particular value, and/or to about another particular value. When such a range is expressed, this includes from the one particular value and/or to the other particular value. Similarly, when values are expressed as approximations, by use of the antecedent about, it will be understood that the particular value is disclosed.

## EXAMPLES

Recombinant proteins have been produced for over thirty years. The goal has been and continues to be to try to produce soluble recombinant proteins. This is particularly true when the aim has been to produce recombinant eukaryotic proteins in bacteria. In general, insoluble recombinant protein has been ignored because insoluble protein has been viewed as being protein inclusion bodies without biological activity. Insoluble protein is also viewed as difficult to renature to active soluble protein with native activity.

However, soluble recombinant proteins are not without problems. The problems with soluble recombinant proteins include increased susceptibility to degradation, difficulty in the separation of desired large recombinant protein from other soluble proteins with possess similar physical characteristics, removal of nucleic acids and the removal of endotoxins. When all of these problems are taken into account, losses of target protein can exceed 95 percent. For difficult to express proteins, where the amount of total recombinant protein express is quite low, then the amount of protein meeting drug standards can be exceptionally low.

Although the methods set forth herein can be used to obtain a heterologous polypeptide, i.e. a recombinant protein, from soluble and insoluble protein fractions, these methods take advantage of the insoluble proteins or inclusion bodies for producing recombinant fusion proteins from host cells, thus resulting in significant increases in the amount of pure target recombinant protein(s) that can be obtained. The process for purifying fusion-proteins is based upon producing insoluble recombinant proteins, which are subsequently renatured to a biologically active state such that removal of endotoxin from the fusion-protein of interest is greatly facilitated and the number of process steps to obtain purified fusion protein is reduced. It is desirable to 1) employ a vector/operon/inducer that increases the amount of insoluble recombinant protein; 2) employ a host, for example a protease negative bacteria, that is compatible with the vector/operon used above; and 3) grow the host and induce expression of the fusion protein.

In the methods set forth herein, fermentation can comprise a growth stage followed by an induction stage. Where it is possible to decouple growth from induction, the amount of recombinant protein will be increased as compared to a combined/growth phase. For example, a fed batch growth technique is employed to obtain high cell density, followed by induction. The cells can then be harvested (for example, by filtration, centrifugation or decanting) and lysed to obtain the recombinant protein. The recombinant protein can be further purified as described herein.

### Materials

The following stock solutions can be utilized for preparing growth media.

Thiamine HCl

10 g of thiamine HCl are dissolved in 950ml dd$H_2O$ and made up to 1L
Trace metal stock solution
5g EDTA
10g $FeSO_4 \cdot 7H_2O$

2g $ZnSO_4 \cdot 7H_2O$
2g $MnSO_4 \cdot H_2O$
0.2g $CoCl_2 \cdot 6H_2O$
0.1g $CuSO_4 \cdot 5H_2O$
0.2g $Na_2MoO_4 \cdot 2H_2O$; and
0.1g $H_3BO_3$ are dissolved in 900ml warm dd$H_2O$.

The solution is then made up to 1L with dd$H_2O$.

Glucose stock solution

250g glucose are dissolved in 700 ml dd$H_2O$. The solution is then made up to 1L with dd$H_2O$ and filter sterilized.

$MgSO_4$ stock solution

78g $MgSO_4$ are dissolved in 900 ml dd$H_2O$. The solution is then made up to 1L with dd$H_2O$ and filter sterilized.

$CaCl_2$ stock solution

80g $CaCl_2$ are dissolved in 950 ml dd$H_2O$. The solution is then made up to 1L with dd$H_2O$ and filter sterilized.

Synthetic Media

Examples of synthetic media and their components are set forth below. For example, the ECAM media that can be utilized for flask work and adaptation can be as follows:

| **ECAM media** | |
|---|---|
| **Component** | **Amount per liter** |
| dd water | 940ml |
| $KH_2PO_4$ | 7.8g |
| Citric acid | 1.0g |
| $(NH_4)_2SO_4$ | 2.33g |
| Trace metal solution | 1ml |
| Thiamine HCl solution | 1ml |
| Glucose stock solution | 40ml |
| $MgSO_4$ solution | 13ml |
| $CaCl_2$ solution | 0.5ml |
| Carbenicillin | 50mg |
| Chloramphenicol | 34mg |
| 10 N NaOH solution | pH to 7.2 |

An example of 10X EBAT media that can be utilized for batch fermentation is set forth below.

| **10X EBAT media** | |
|---|---|
| **Component** | **Amount/liter** |
| antifoam | 750 μl |
| $KH_2PO_4$ | 22g |
| Citric acid | 10g |
| $(NH_4)_2SO_4$ | 45g |

(continued)

| 10X EBAT media | |
|---|---|
| **Component** | **Amount/liter** |
| Trace metal solution | 10ml |
| Thiamine HCl solution | 10ml |
| Make up to 1.5 liter and autoclave | |

An example of 10X EBATII media that can be utilized for batch fermentation is set forth below.

| 10X EBAT II- media | |
|---|---|
| **Component** | **Amount/liter** |
| Glucose stock solution | 875ml |
| $MgSO_4$ solution | 130ml |
| $CaCl_2$ solution | 5ml |
| Carbenicillin | 0.5g |
| Chloramphenicol | 0.34g |
| Make up to 2.0 liter and filter sterilize | |

An example of EFED feed that can be utilized for fed batch fermentation is set forth below.

| EFED- feed for fermenter | |
|---|---|
| **Component** | **Amount/liter** |
| Dextrose | 180 g |
| L-Alanine | 40g |
| $KH_2PO_4$ | 1.5g |
| $(NH_4)_2HPO_4$ | 10 g |
| $NaH_2PO_4$ | 4 |
| $(NH_4)_2SO_4$ | 5g |
| Citric acid | 4 g |
| pH to 5.2 with Sulfuric acid | |
| $MgSO_4$ | 30ml |
| Trace metals | 6 ml |
| $CaCl_2$ solution | 25 ml |
| Thiamine HCl | 1 ml |
| $FeSO_4$ solution | 25 ml |
| Make up to 1L with dd water pH to 6 with 10 N NaOH | |

Growth conditions

Once the host cells, for example, *E. coli* BL21 DE3 Tuner® cells, comprising a nucleic acid encoding the heterologous polypeptide have been confirmed and validated to contain the desired insert sequence and to exhibit induction with 1 mM IPTG they enter into the fermentation stage. Prior to fermentation the clones are adapted. This is done by growing the clone(s) in ECAM media described above. Following adaptation, glycerol stocks are generated that are used for initiating the inoculums for the fermenter. Fermentation can be conducted in two phases. Phase 1 is a batch fermentation phase followed by a fed batch fermentation phase in Phase 2.

Phase 1A: Adaptation to fermentation media (ECAM)

One glycerol stock (cells in LB media) was transferred to 200 ml of LB media in a 500 ml flask. The culture was incubated overnight at 37°C at 200 RPM. When the culture was between an OD of 1-1.2, 5 ml of the culture was transferred to 250 ml ECAM in a 1L flask. This culture was incubated at 37°C at 200 RPM. After 6-7 hrs when the OD was 1-1.2, 5 ml of the culture was transferred to 250 ml ECAM in a 1 L flask and incubated overnight at 37°C at 200 RPM. When the OD was between 1-1.2, 30% glycerol stocks (1ml aliquots) were prepared of the adapted cells.

Phase 1B: Flask work for fermenter

Two glycerol stock vials were transferred to 250ml ECAM in a 1L flask. The culture was incubated at 37°C at 200 RPM overnight. At an OD of 1-1.2, 5ml of the culture was transferred to 250ml ECAM in a 1L flask and grown for 7 hrs. This was used as inoculum for the fermentation vessel. The vessel was inoculated to give 1.5 X $10^{-2}$ AU/L (absorbance units/liter).

Phase 2A: Batch Fermentation

Conventional 20L or 30 L reactors were used for batch fermentation. Phase 2A batch volumes were 10L and 15L for 20 and 30L reactors, respectively. Fermentation was carried out in EBAT-I or EBAT-II media at 30 °C. EBAT-1 is sterilized by autoclaving while EBAT-II is sterilized by filtration. The pH was maintained at 7, dissolved oxygen was maintained at 30% using the stirrer and gas mix control loops. Initial glucose concentration was 20g/L.

Phase 2B: Fed-Batch Fermentation

The addition of 5L or 8.5L feed (EFED media) started after glucose exhaustion for 20 L and 30 L reactors respectively. Maximum feed rate varies depending on the construct. Once maximum feed rate is achieved IPTG is added to give a final concentration of 1.2mM.

Cell Disruption and Clarification

After batch fermentation, the bacterial cells in the synthetic media can be harvested, for example, be decanting. The bacterial cell pellet or paste formed after decanting can be further processed or frozen for further processing later. The cells can be disrupted and clarified as follows. For example, frozen cell paste was placed at 4°C to thaw overnight. Lysis buffer (50mM Tris, 125mM NaCl, 4% Sucrose, 10mM EDTA, 0.05% TritonX-100 at pH 8)) was chilled at 4°C overnight. The cell paste was then transferred to a volumetric mixing container with the capacity to hold up to 2L. Lysis buffer was added until the total volume of cell paste plus lysis buffer was 1L. The lysis buffer/cell paste was mixed on a stir plate for at least 15 minutes. The resuspended cell paste was placed in the refrigerator or an ice bath to cool to <5°C.

The resuspended cell paste was homogenized using an APV homogenizer. The fill feed hopper of the APV homogenizer was filled with resuspended cell paste. The sample was run through the APB at 0 psi (0 bar). The resuspended cell paste was cooled to < 5°C. Then 1) the fill feed hopper was filled with the resuspended cell paste; 2) the sample was run through the APV at 10,000 psi (690bar); and 3) the lysate was cooled to < 5°C. Steps 1-3 were repeated until the lysate had been homogenized 3 times under pressure. After the final pass, the lysate was collected and cooled to 14°C. The lysate was then clarified by centrifugation at 8,000 RPM for 30 minutes.. After centrifugation, pellets were resuspended in equal volumes of buffer (50mM phosphate, 200mM NaCl, 10mM EDTA, and 1% tritonX-100 at pH 7) by vortexing and centrifuging for 30 min at 8,000 RPM. The supernatant was discarded and the pellet was rinsed with buffer (50mM Tris, 125mM NaCl, 10mM EDTA, pH 8) to remove detergent. The resuspension, centrifuging and rinsing step were repeated as necessary. Pellets were resuspended in equal volumes of 0.3M Urea buffer by swirling and centrifuged for 30min at 8,000 RPM. A sample of the supernatant was obtained before discarding the supernatant. If not processed immediately, the pellet was stored at -20°C.

The pellet was then resuspended in P4 buffer (100mM tris, 6M urea at pH 8). The resuspended pellet was stirred for at least 15 minutes and then centrifuged at room temperature for 60 minutes at 8000 rpm. After centrifugation, the supernatants were collected and pooled. The total volume of the pooled supernatants was measured. The pellets were pooled and weighed. The pellets comprising the inclusion bodies were then processed to allow solubilization and refolding of the heterologous polypeptide by using the Novagen Protein Refolding Kit (SP No. 70123-3) according to the manufacturer's instructions. After refolding of the polypeptide, anion exchange chromatography was used to remove any remaining bacterial cell protein/endotoxin, misfolded proteins and buffer components.

Anion Exchange Column - Denatured Load and Renaturing

Preparation

1.Determine protein concentration of the filtered column load (FCL), for example, by using a BCA protein assay.
2.Denature FCL

a) Obtain FCL, record volume, conductivity, and pH
b) Raise temperature of FCL to 15-25°C, record temperature
c) Slowly add required amount of urea pellets to achieve final 8M concentration. (Urea required = 765g urea/volume of FCL (L)
d) Stir for 2 hours

3.Dilute FCL (loading of anion exchange column)

Use a commercially prepared resin in a column or use resin to make a column.

a) Estimate protein concentration of FCL
Either directly measure via BCA protein assay (using a 1:1dilution of sample) or calculate based on original [FCL] and new total volume
b) Use a Buffer P5 (100mM Tris, 8M urea, pH8), make up a 2mg/mL dilution of FCL (final volume = 50mL)
c) Calculate and record final urea concentration

Determine Final concentration of Urea= [(Urea added, kg *60.0 6) + (amount Buffer P5 added, L X 8)/Final volume denatured FCL

d) Measure and record pH and conductivity of Denatured Q
e) This is the Denatured Column Load (DCL)

Refolding

1. Record pH, conductivity, and batch information of refolding buffer, for example, a buffer comprising 20mM Tris, 0.5M Urea, 0.1M Trehalose, 2mM $CaCL_2$, 3mM Cysteine, 0.3mM Cystine, 1mM EDTA, 0.1% PS-80, pH 8.0. Numerous commercially available refolding buffers can be used.
2. Calculate volume of DCL containing at least 50mg protein (~25mL)
3. Add required volume of Refolding Buffer to clean bottle

a) Volume Refolding Buffer = 9*Volume of DCL

4.Slowly add DCL (5Ml/min) to Refolding Buffer and stir for ~20min. The refolding step can be continued for more than 20 minutes, for example, this step can be continued for several hours or overnight.
5. Measure and record pH and conductivity of Renatured Column Load (RCL)

Load and Run

1. Direct line from RCL to sample valve of HPLC.
2. Run standard column elution protocol
3. Collect post load wash, elutions, strip, and load flow through

4. Once run is completed flush lines with ddH20 and 20% Ethanol

Construct Design

To obtain S. *typhimurium* DNA contain *fliC-DNA* the Qiagen® DNeasy Kit was used according to_the manufacturer's specified protocol for DNA extraction from blood and tissue. To obtain the *flic* amplicon the DNA sequence was first identified using the National Center for Biotechnology Information, U.S. National Library of_Medicine (NCBI) Database using the Basic Local Alignment and Search Tool (BLAST). The sequence information was then used to design primers. The primers were_designed using Integrated DNA Technologies (IDT) SciTools PrimerQuest® primer design software to make primers specific for *fliC.* PCR was utilized to obtain a fliC amplicon from of S. *typhimurium* DNA. The amplicon was separated on 1.2% agarose with GelRed (Biotium) to visualize the DNA bands. The PCR product was excised from the 1.2% agarose gel using the Qiagen Qiaquick Gel Extraction Kit®. Ligation of the FliC amplicon into the IPTG inducible plasmid pETBlue (commercially available from Novagen) was performed using the Novagen Perfectly Blunt Cloning kit. The Novagen protocol for ligation (Novagen pET System Manual) was modified to include a 2 hour ligation period at a ligation temperature of 16°C. Positive transcription hosts were identified and the plasmids were excised according to the Qiagen Plasmid Mini kit. The plasmids were analyzed by DNA sequencing to_determine proper orientation of the insert using the pETBlue up and pETBlue down primers that are commercially available from Novagen. The plasmids (containing full-length FliC in the proper orientation) were transformed into the expression host cell from Novagen BL21(DE3)Tuner cells using the Novagen Perfectly Blunt Cloning Kit.

Utilizing standard cloning techniques, a vector comprising a nucleotide sequence encoding the full length S. *typhimurium* type 2 flagellin (STF2) and a globular head subunit of influenza hemagglutinin (HA1-2) was constructed. The construct was expressed in *E. coli* utilizing the methods described herein and the heterologous polypeptide (STF2-HA1 fusion protein) was obtained. The recombinant protein STF2-HA1 was obtained in yields of about 3700 mg/L. The methods set forth herein can produce yields of 3-10g/L. The resulting purified STF2-HA1 elicited a strong immune response that was effective at a dose of less than 1 microgram.

In another example, utilizing standard cloning techiniques, a vector comprising a nucleotide sequence encoding fliC flagellin (amino acids 1-465 of SEQ ID NO: 1) and an antigenic portion (SEQ ID NO: 5) of a Marburg virus glycoprotein, gp132 was constructed. The nucleotide sequence encoding the amino acid sequence of the gp132-fliC fusion protein is set forth herein as SEQ ID NO: 8. The amino acid sequence of the gp132-fliC fusion protein is set forth herein as SEQ ID NO: 9. The construct was expressed in *E. coli* utilizing the methods described herein and the heterologous polypeptide (gp132-FliC fusion protein) was obtained. The recombinant fusion protein gp132-FliC was obtained in yields of about 5 g/L. Western blots also showed that the fusion protein had FliC activity.

## Claims

**1.** A method of producing a heterologous polypeptide, the method comprising the steps of:

a) growing Gram-negative bacteria comprising a nucleotide sequence encoding a heterologous polypeptide operably linked to an inducible promoter under fed-batch fermentation conditions in a synthetic medium;
b) inducing expression of the heterologous polypeptide;
c) harvesting the bacteria in the synthetic medium by decanting;
d) homogenizing the bacteria contained in the synthetic medium;
e) obtaining from the synthetic medium comprising the homogenized bacteria of step d) a soluble fraction comprising the heterologous polypeptide and an insoluble fraction comprising the heterologous polypeptide;
f) resuspending the insoluble fraction;
g) centrifuging the resuspended insoluble fraction of step g) to obtain a supernatant comprising the heterologous polypeptide;
h) denaturing the polypeptide in the supernatant obtained from step g);
i) refolding the denatured polypeptide of step h); and
j) subjecting the refolded polypeptide to anion exchange chromatography to obtain the heterologous polypeptide.

**2.** The method of claim 1, further comprising the steps of:

k) precipitating the heterologous polypeptide from the soluble fraction of step e) in a precipitate;
l) resuspending the polypeptide precipitate from step k);
m) centrifuging the resuspended polypeptide precipitate to obtain a supernatant comprising the heterologous polypeptide;
n) subjecting the supernatant obtained in step m) to tangential flow filtration to form a filtration product;
o) performing a two-phase separation on the filtration product of step n) to produce an aqueous polypeptide phase and a detergent phase; p) denaturing the polypeptide in the aqueous polypeptide phase obtained in step o);
q) refolding the denatured polypeptide of step p); and
r) subjecting the refolded polypeptide to anion exchange chromatography to obtain the heterologous polypeptide.

**3.** The method of claim 2, wherein the soluble fraction and insoluble fraction are obtained by centrifuging the synthetic medium comprising the homogenized bacteria of step d).

**4.** The method of claim 1 , further comprising subjecting the heterologous polypeptide of step j) to HIC chromatography.

**5.** The method of claim 2, further comprising subjecting the heterologous polypeptide of step r) to HIC chromatography.

**6.** The method of claim 1 or 2, wherein the bacteria is adapted to synthetic medium, prior to step a).

**7.** The method of any one of claims 1 to 6, wherein a vector comprises the nucleotide sequence encoding heterologous polypeptide operably linked to an inducible promoter.

**8.** The method of any one of claims 1 to 7, wherein the bacteria is E. coli.

**9.** The method of any one of claims 1 to 8, wherein the expression of the heterologous polypeptide is induced by addition of IPTG to the medium.

**10.** The method of any one of claims 1 to 9, wherein the heterologous polypeptide is a fusion protein comprising a flagellin or a fragment thereof and at least one antigenic peptide.

**11.** The method of claim 10, wherein

(i) the peptide is linked to the N-terminus of a flagellin or a fragment thereof; and/or
(ii) the peptide is linked to the C-terminus of a flagellin or a fragment thereof; and/or
(iii) the N-terminus and the C-terminus of the peptide are linked to a flagellin or a fragment thereof.

**12.** The method of claim 10 or claim 11 wherein the flagellin is a Flic flagellin.

**13.** The method of any one of claims 10 to 12, wherein the peptide is a fragment from a bacterial, viral, parasitic or fungal protein.

**14.** The method of claim 13, wherein the antigenic peptide elicits antibodies against a bacteria, a virus, a parasite or a fungus.

**15.** The method of any one of claims 10 to 14, wherein

(i) the peptide comprises from about 10 amino acids to about 25 amino acids; or
(ii) the peptide comprises from about 25 amino acids to about 50 amino acids; or
(iii) the peptide comprises from about 50 amino acids to about 100 amino acids; or
(iv) the peptide comprises from about 100 amino acids to about 200 amino acids; or
(v) the peptide comprises from about 200 amino acids to about 400 amino acids.

**Patentansprüche**

**1.** Verfahren zur Herstellung eines heterologen Polypeptids, wobei das Verfahren folgende Schritte umfasst:

a) Züchten gram-negativer Bakterien, die eine Nukleotidsequenz umfassen, die für ein heterologes Polypeptid codiert, das unter Fed-Batch-Fermentationsbedingungen in einem synthetischen Medium funktionsfähig mit einem induzierbaren Promoter verknüpft wird;
b) Induzieren einer Expression des heterologen Polypeptids;
c) Ernten der Bakterien in dem synthetischen Medium durch Dekantieren;
d) Homogenisieren der in dem synthetischen Medium enthaltenen Bakterien;
e) Erzielen einer löslichen Fraktion, welche das heterologe Polypeptid umfasst, und einer nicht-löslichen Fraktion, die das heterologe Polypeptid umfasst, aus dem synthetischen Medium, das die homogenisierten Bakterien von Schritt d) umfasst;
f) Resuspendieren der nicht-löslichen Fraktion;
g) Zentrifugieren der resuspendierten nicht-löslichen Fraktion von Schritt g), um einen Überstand zu erzielen, der das heterologe Polypeptid umfasst;
h) Denaturieren des Polypeptids in dem in Schritt g) erzielten Überstand;
i) Rückfalten des denaturierten Polypeptids von Schritt h) und
j) Unterziehen des rückgefalteten Polypeptids einer Anionenaustauschchromatografie, um das heterologe Polypeptid zu erzielen.

**2.** Verfahren nach Anspruch 1, ferner folgende Schritte umfassend:

k) Ausfällen des heterologen Polypeptids aus der löslichen Fraktion von Schritt e) in einer Ausfällung;
l) Resuspendieren der Polypeptidausfällung von Schritt k);
m) Zentrifugieren der resuspendierten Polypeptidausfällung, um einen Überstand zu erzielen, der das heterologe Polypeptid umfasst;
n) Unterziehen des in Schritt m) erzielten Überstandes einer Tangentialflussfiltration, um ein Filtrationsprodukt zu bilden;
o) Ausführen einer Zweiphasenabscheidung an dem Filtrationsprodukt von Schritt n), um eine wässrige Polypeptidphase und eine Detergentphase herzustellen;
p) Denaturieren des Polypeptids in der in Schritt o) erzielten wässrigen Polypeptidphase;
q) Rückfalten des denaturierten Polypeptids von Schritt p) und
r) Unterziehen des rückgefalteten Polypeptids einer Anionenaustauschchromatografie, um das heterologe Polypeptid zu erzielen.

**3.** Verfahren nach Anspruch 2, wobei die lösliche Fraktion und die nicht-lösliche Fraktion durch Zentrifugieren des synthetischen Mediums erzielt werden, das die homogenisierten Bakterien von Schritt d) umfasst.

**4.** Verfahren nach Anspruch 1, ferner das Unterziehen des heterologen Polypeptids von Schritt j) der HIC-Chromatografie umfassend.

**5.** Verfahren nach Anspruch 2, ferner das Unterziehen des heterologen Polypeptids von Schritt r) der HIC-Chromatografie umfassend.

**6.** Verfahren nach Anspruch 1 oder 2, wobei das Bakterium vor Schritt a) synthetischem Medium angepasst wird.

**7.** Verfahren nach einem der Ansprüche 1 bis 6, wobei ein Vektor die Nukleotidsequenz umfasst, die für das heterologe Polypeptid codiert, das funktionsfähig mit einem induzierbaren Promoter verknüpft wird.

**8.** Verfahren nach einem der Ansprüche 1 bis 7, wobei das Bakterium E. coli ist.

**9.** Verfahren nach einem der Ansprüche 1 bis 8, wobei die Expression des heterologen Polypeptids durch das Zusetzen von IPTG zum Medium induziert wird.

**10.** Verfahren nach einem der Ansprüche 1 bis 9, wobei das heterologe Polypeptid ein Fusionsprotein ist, das ein Flagellin oder ein Fragment davon und mindestens ein antigenes Peptid umfasst.

**11.** Verfahren nach Anspruch 10, wobei:

(I) das Peptid mit dem N-Terminus eines Flagellins oder eines Fragments davon verknüpft ist und/oder
(II) das Peptid mit dem C-Terminus eines Flagellins oder eines Fragments davon verknüpft ist und/oder
(III) der N-Terminus und der C-Terminus des Peptids mit einem Flagellin oder einem Fragment davon verknüpft sind.

**12.** Verfahren nach Anspruch 10 oder Anspruch 11, wobei das Flagellin ein FliC-Flagellin ist.

**13.** Verfahren nach einem der Ansprüche 10 bis 12, wobei das Peptid ein Fragment von einem bakteriellen, viralen oder Pilzprotein ist.

**14.** Verfahren nach Anspruch 13, wobei das antigene Peptid Antikörper gegen ein Bakterium, ein Virus, einen Parasiten oder einen Pilz hervorruft.

**15.** Verfahren nach einem der Ansprüche 10 bis 14, wobei:

(I) das Peptid etwa 10 Aminosäuren bis etwa 20 Aminosäuren umfasst oder
(II) das Peptid etwa 25 Aminosäuren bis etwa 50 Aminosäuren umfasst oder
(III) das Peptid etwa 50 Aminosäuren bis etwa 100 Aminosäuren umfasst oder
(IV) das Peptid etwa 100 Aminosäuren bis etwa 200 Aminosäuren umfasst oder
(V) das Peptid etwa 200 Aminosäuren bis etwa 400 Aminosäuren umfasst.

## Revendications

**1.** Procédé de production d'un polypeptide hétérologue, le procédé comprenant les étapes consistant à :

a) faire croître des bactéries à Gram négatif comprenant une séquence de nucléotides codant pour un polypeptide hétérologue en liaison fonctionnelle avec un promoteur inductible dans des conditions de fermentation discontinue alimentée dans un milieu synthétique ;
b) induire l'expression du polypeptide hétérologue ;
c) récolter les bactéries dans le milieu synthétique par décantation ;
d) homogénéiser les bactéries contenues dans le milieu synthétique ;
e) obtenir, à partir du milieu synthétique comprenant les bactéries homogénéisées de l'étape d), une fraction soluble comprenant le polypeptide hétérologue et une fraction insoluble comprenant le polypeptide hétérologue ;
f) remettre en suspension la fraction insoluble ;

g) centrifuger la fraction insoluble remise en suspension de l'étape g) afin d'obtenir un surnageant comprenant le polypeptide hétérologue ;
h) dénaturer le polypeptide dans le surnageant obtenu à l'étape g) ;
i) replier le polypeptide dénaturé de l'étape h) ; et
j) soumettre le polypeptide replié à une chromatographie échangeuse d'anions afin d'obtenir le polypeptide hétérologue.

**2.** Procédé selon la revendication 1, comprenant en outre les étapes consistant à :

k) précipiter le polypeptide hétérologue à partir de la fraction soluble de l'étape e) dans un précipité ;
l) remettre en suspension le précipité de polypeptide de l'étape k) ;
m) centrifuger le précipité de polypeptide remis en suspension afin d'obtenir un surnageant comprenant le polypeptide hétérologue ;
n) soumettre le surnageant obtenu à l'étape m) à une filtration à flux tangentiel afin de former un produit de filtration ;
o) réaliser une séparation en deux phases sur le produit de filtration de l'étape n) afin de produire une phase de polypeptide aqueuse et une phase de détergent ;
p) dénaturer le polypeptide dans la phase de polypeptide aqueuse obtenue à l'étape o) ;
q) replier le polypeptide dénaturé de l'étape p) ; et
r) soumettre le polypeptide replié à une chromatographie échangeuse d'anions afin d'obtenir le polypeptide hétérologue.

**3.** Procédé selon la revendication 2, dans lequel la fraction soluble et la fraction insoluble sont obtenues par une centrifugation du milieu synthétique comprenant les bactéries homogénéisées de l'étape d).

**4.** Procédé selon la revendication 1, comprenant en outre le fait de soumettre le polypeptide hétérologue de l'étape j) à une chromatographie HIC.

**5.** Procédé selon la revendication 2, comprenant en outre le fait de soumettre le polypeptide hétérologue de l'étape r) à une chromatographie HIC.

**6.** Procédé selon la revendication 1 ou 2, dans lequel les bactéries sont adaptées au milieu synthétique, avant l'étape a).

**7.** Procédé selon l'une quelconque des revendications 1 à 6, dans lequel un vecteur comprend la séquence de nucléotides codant pour le polypeptide hétérologue en liaison fonctionnelle avec un promoteur inductible.

**8.** Procédé selon l'une quelconque des revendications 1 à 7, dans lequel les bactéries sont E. coli.

**9.** Procédé selon l'une quelconque des revendications 1 à 8, dans lequel l'expression du polypeptide hétérologue est induite par l'addition d'IPTG au milieu.

**10.** Procédé selon l'une quelconque des revendications 1 à 9, dans lequel le polypeptide hétérologue est une protéine de fusion comprenant une flagelline ou un fragment de celle-ci et au moins un peptide antigénique.

**11.** Procédé selon la revendication 10, dans lequel

(i) le peptide est lié à l'extrémité N-terminale d'une flagelline ou d'un fragment de celle-ci ; et/ou
(ii) le peptide est lié à l'extrémité C-terminale d'une flagelline ou d'un fragment de celle-ci ; et/ou
(iii) l'extrémité N-terminale et l'extrémité C-terminale du peptide sont liées à une flagelline ou un fragment de celle-ci.

**12.** Procédé selon la revendication 10 ou la revendication 11, dans lequel la flagelline est une flagelline Flic.

**13.** Procédé selon l'une quelconque des revendications 10 à 12, dans lequel le peptide est un fragment d'une protéine bactérienne, virale, parasitaire ou fongique.

**14.** Procédé selon la revendication 13, dans lequel le peptide antigénique génère des anticorps dirigés contre une bactérie, un virus, un parasite ou un champignon.

**15.** Procédé selon l'une quelconque des revendications 10 à 14, dans lequel

(i) le peptide comprend entre environ 10 acides aminés et environ 25 acides aminés ; ou
(ii) le peptide comprend entre environ 25 acides aminés et environ 50 acides aminés ; ou
(iii) le peptide comprend entre environ 50 acides aminés et environ 100 acides aminés ; ou
(iv) le peptide comprend entre environ 100 acides aminés et environ 200 acides aminés ; ou
(v) le peptide comprend entre environ 200 acides aminés et environ 400 acides aminés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61513281 B

### Non-patent literature cited in the description


- **CRASTO ; FENG.** LINKER: a program to generate linker sequences for fusion proteins. *PEDS,* 2000, vol. 13 (5), 309-312
- **RUSSELL et al.** The global circulation of seasonal influenza A (H3N2) viruses. *Science,* 2008, vol. 320 (5874), 340-346
- **CHAIPAN et al.** *Proteolytic activation of the 1918 Influenza Virus Hemagglutinin Journal of Virology,* 2009, vol. 83 (7), 3200-3211
- **KALINA et al.** Discovery of common marburgvirus protective epitopes in BALB/c mouse model. *Virology Journal,* 2009, vol. 6, 132
- **CAO et al.** Purification of clinical-grade recombinant HSP65-MUCI fusion protein. *Biotechnol. Appl. Biochem.,* 2010, vol. 57, 9-15
- **BHUVANESH et al.** Production and single-step purification of Brugia malayi abundant larval transcript (ALT-2) using hybdrophobic interaction chromatography. *J. Ind. Microbl. Biotechnol.,* 2010, vol. 37 (10), 1053-9
- Remington's Pharmaceutical Sciences. University of the Sciences in Philadelphia, Lippincott, Williams & Wilkins, 2005

SEQUENCE LISTING

<110> Georgia State University Research Foundation
Pierce, George

<120> Protein Production Method

<130> 10013-017WO1

<150> US 61/513,281
<151> 2011-07-29

<160> 9

<170> PatentIn version 3.5

<210> 1
<211> 495
<212> PRT
<213> Salmonella typhimurium

<400> 1

```
Met Ala Gln Val Ile Asn Thr Asn Ser Leu Ser Leu Leu Thr Gln Asn
1               5                   10                  15

Asn Leu Asn Lys Ser Gln Ser Ala Leu Gly Thr Ala Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ala Asn Ile Lys Gly Leu Thr Gln Ala
    50                  55                  60

Ser Arg Asn Ala Asn Asp Gly Ile Ser Ile Ala Gln Thr Thr Glu Gly
65                  70                  75                  80

Ala Leu Asn Glu Ile Asn Asn Asn Leu Gln Arg Val Arg Glu Leu Ala
                85                  90                  95

Val Gln Ser Ala Asn Ser Thr Asn Ser Gln Ser Asp Leu Asp Ser Ile
            100                 105                 110

Gln Ala Glu Ile Thr Gln Arg Leu Asn Glu Ile Asp Arg Val Ser Gly
        115                 120                 125

Gln Thr Gln Phe Asn Gly Val Lys Val Leu Ala Gln Asp Asn Thr Leu
    130                 135                 140

Thr Ile Gln Val Gly Ala Asn Asp Gly Glu Thr Ile Asp Ile Asp Leu
145                 150                 155                 160
```

```
Lys Gln Ile Asn Ser Gln Thr Leu Gly Leu Asp Thr Leu Asn Val Gln
                165                 170                 175

Gln Lys Tyr Lys Val Ser Asp Thr Ala Ala Thr Val Thr Gly Tyr Ala
            180                 185                 190

Asp Thr Thr Ile Ala Leu Asp Asn Ser Thr Phe Lys Ala Ser Ala Thr
        195                 200                 205

Gly Leu Gly Gly Thr Asp Gln Lys Ile Asp Gly Asp Leu Lys Phe Asp
    210                 215                 220

Asp Thr Thr Gly Lys Tyr Tyr Ala Lys Val Thr Val Thr Gly Gly Thr
225                 230                 235                 240

Gly Lys Asp Gly Tyr Tyr Glu Val Ser Val Asp Lys Thr Asn Gly Glu
                245                 250                 255

Val Thr Leu Ala Gly Gly Ala Thr Ser Pro Leu Thr Gly Gly Leu Pro
            260                 265                 270

Ala Thr Ala Thr Glu Asp Val Lys Asn Val Gln Val Ala Asn Ala Asp
        275                 280                 285

Leu Thr Glu Ala Lys Ala Ala Leu Thr Ala Ala Gly Val Thr Gly Thr
    290                 295                 300

Ala Ser Val Val Lys Met Ser Tyr Thr Asp Asn Asn Gly Lys Thr Ile
305                 310                 315                 320

Asp Gly Gly Leu Ala Val Lys Val Gly Asp Asp Tyr Tyr Ser Ala Thr
                325                 330                 335

Gln Asn Lys Asp Gly Ser Ile Ser Ile Asn Thr Thr Lys Tyr Thr Ala
            340                 345                 350

Asp Asp Gly Thr Ser Lys Thr Ala Leu Asn Lys Leu Gly Gly Ala Asp
        355                 360                 365

Gly Lys Thr Glu Val Val Ser Ile Gly Gly Lys Thr Tyr Ala Ala Ser
    370                 375                 380

Lys Ala Glu Gly His Asn Phe Lys Ala Gln Pro Asp Leu Ala Glu Ala
385                 390                 395                 400

Ala Ala Thr Thr Thr Glu Asn Pro Leu Gln Lys Ile Asp Ala Ala Leu
                405                 410                 415
```

```
Ala Gln Val Asp Thr Leu Arg Ser Asp Leu Gly Ala Val Gln Asn Arg
            420                 425                 430

Phe Asn Ser Ala Ile Thr Asn Leu Gly Asn Thr Val Asn Asn Leu Thr
        435                 440                 445

Ser Ala Arg Ser Arg Ile Glu Asp Ser Asp Tyr Ala Thr Glu Val Ser
    450                 455                 460

Asn Met Ser Arg Ala Gln Ile Leu Gln Gln Ala Gly Thr Ser Val Leu
465                 470                 475                 480

Ala Gln Ala Asn Gln Val Pro Gln Asn Val Leu Ser Leu Leu Arg
                485                 490                 495
```

<210> 2
<211> 1488
<212> DNA
<213> Salmonella typhimurium

<400> 2

```
atggcacaag tcattaatac aaacagcctg tcgctgttga cccagaataa cctgaacaaa      60
tcccagtccg ctctgggcac cgctatcgag cgtctgtctt ccggtctgcg tatcaacagc     120
gcgaaagacg atgcggcagg tcaggcgatt gctaaccgtt ttaccgcgaa catcaaaggt     180
ctgactcagg cttcccgtaa cgctaacgac ggtatctcca ttgcgcagac cactgaaggc     240
gcgctgaacg aaatcaacaa caacctgcag cgtgtgcgtg aactggcggt tcagtctgct     300
aacagcacca actcccagtc tgacctcgac tccatccagg ctgaaatcac ccagcgcctg     360
aacgaaatcg accgtgtatc cggccagact cagttcaacg gcgtgaaagt cctggcgcag     420
gacaacaccc tgaccatcca ggttggtgcc aacgacggtg aaactatcga tatcgatctg     480
aagcagatca actctcagac cctgggtctg gatacgctga atgtgcaaca aaaatataag     540
gtcagcgata cggctgcaac tgttacagga tatgccgata ctacgattgc tttagacaat     600
agtactttta aagcctcggc tactggtctt ggtggtactg accagaaaat tgatggcgat     660
ttaaaatttg atgatacgac tggaaaatat tacgccaaag ttaccgttac gggggggaact    720
ggtaaagatg gctattatga agtttccgtt gataagacga acggtgaggt gactcttgct     780
ggcggtgcga cttccccgct tacaggtgga ctacctgcga cagcaactga ggatgtgaaa     840
aatgtacaag ttgcaaatgc tgatttgaca gaggctaaag ccgcattgac agcagcaggt     900
gttaccggca cagcatctgt tgttaagatg tcttatactg ataataacgg taaaactatt     960
gatggtggtt tagcagttaa ggtaggcgat gattactatt ctgcaactca aaataaagat    1020
ggttccataa gtattaatac tacgaaatac actgcagatg acggtacatc caaaactgca    1080
```

```
ctaaacaaac tgggtggcgc agacggcaaa accgaagttg tttctattgg tggtaaaact     1140

tacgctgcaa gtaaagccga aggtcacaac tttaaagcac agcctgatct ggcggaagcg     1200

gctgctacaa ccaccgaaaa cccgctgcag aaaattgatg ctgctttggc acaggttgac     1260

acgttacgtt ctgacctggg tgcggtacag aaccgtttca actccgctat taccaacctg     1320

ggcaacaccg taaacaacct gacttctgcc cgtagccgta tcgaagattc cgactacgcg     1380

accgaagttt ccaacatgtc tcgcgcgcag attctgcagc aggccggtac ctccgttctg     1440

gcgcaggcga accaggttcc gcaaaacgtc ctctctttac tgcgttaa                 1488
```

<210> 3
<211> 15
<212> PRT
<213> Artificial Sequence

<220>
<223> Synthetic Construct

<400> 3

```
Ile Leu Ile Gln Gly Thr Lys Asn Leu Pro Ile Leu Glu Ile Ala
1               5                   10                  15
```

<210> 4
<211> 15
<212> PRT
<213> Artificial Sequence

<220>
<223> Synthetic Construct

<400> 4

```
Glu Pro His Tyr Ser Pro Leu Ile Leu Ala Leu Lys Thr Leu Glu
1               5                   10                  15
```

<210> 5
<211> 15
<212> PRT
<213> Artificial Sequence

<220>
<223> Synthetic Construct

<400> 5

```
Gly Ile Leu Leu Leu Leu Ser Ile Ala Val Leu Ile Ala Leu Ser
1               5                   10                  15
```

<210> 6
<211> 329
<212> PRT
<213> Influenza virus

<400> 6

```
Gln Lys Leu Pro Gly Asn Asp Asn Ser Thr Ala Thr Leu Cys Leu Gly
1               5                   10                  15

His His Ala Val Pro Asn Gly Thr Ile Val Lys Thr Ile Thr Asn Asp
            20                  25                  30

Gln Ile Glu Val Thr Asn Ala Thr Glu Leu Val Gln Ser Ser Ser Thr
        35                  40                  45

Gly Gly Ile Cys Asp Ser Pro His Gln Ile Leu Asp Gly Glu Asn Cys
    50                  55                  60

Thr Leu Ile Asp Ala Leu Leu Gly Asp Pro Gln Cys Asp Gly Phe Gln
65                  70                  75                  80

Asn Lys Lys Trp Asp Leu Phe Val Glu Arg Ser Lys Ala Tyr Ser Asn
                85                  90                  95

Cys Tyr Pro Tyr Asp Val Pro Asp Tyr Ala Ser Leu Arg Ser Leu Val
            100                 105                 110

Ala Ser Ser Gly Thr Leu Glu Phe Asn Asn Glu Ser Phe Asn Trp Thr
        115                 120                 125

Gly Val Thr Gln Asn Gly Thr Ser Ser Ala Cys Lys Arg Arg Ser Asn
    130                 135                 140

Lys Ser Phe Phe Ser Arg Leu Asn Trp Leu Thr His Leu Lys Phe Lys
145                 150                 155                 160

Tyr Pro Ala Leu Asn Val Thr Met Pro Asn Asn Glu Lys Phe Asp Lys
                165                 170                 175

Leu Tyr Ile Trp Gly Val His His Pro Gly Thr Asp Asn Asp Gln Ile
            180                 185                 190

Ser Leu Tyr Ala Gln Ala Ser Gly Arg Ile Thr Val Ser Thr Lys Arg
        195                 200                 205

Ser Gln Gln Thr Val Ile Pro Asn Ile Gly Ser Arg Pro Arg Val Arg
    210                 215                 220

Asp Val Pro Ser Arg Ile Ser Ile Tyr Trp Thr Ile Val Lys Pro Gly
225                 230                 235                 240

Asp Ile Leu Leu Ile Asn Ser Thr Gly Asn Leu Ile Ala Pro Arg Gly
                245                 250                 255
```

```
Tyr Phe Lys Ile Arg Ser Gly Lys Ser Ser Ile Met Arg Ser Asp Ala
            260                 265                 270

Pro Ile Gly Lys Cys Asn Ser Glu Cys Ile Thr Pro Asn Gly Ser Ile
        275                 280                 285

Pro Asn Asp Lys Pro Phe Gln Asn Val Asn Arg Ile Thr Tyr Gly Ala
    290                 295                 300

Cys Pro Arg Tyr Val Lys Gln Asn Thr Leu Lys Leu Ala Thr Gly Met
305                 310                 315                 320

Arg Asn Val Pro Glu Lys Gln Thr Arg
                325
```

<210> 7
<211> 987
<212> DNA
<213> Influenza virus

<400> 7

```
caaaaacttc ccggaaatga caacagcacg gcaacgctgt gccttgggca ccatgcagta       60
ccaaacggaa cgatagtgaa aacaatcacg aatgaccaaa ttgaagtcac taatgctact      120
gagctggttc agagttcctc aacaggtgga atatgcgaca gtcctcatca gatccttgat      180
ggagaaaact gcacactaat agatgctcta ttgggagacc ctcagtgtga tggcttccaa      240
aataagaaat gggacctttt tgttgagcgc agcaaagcct acagcaactg ttacccttat      300
gatgtgccgg attatgcctc ccttaggtca ctagttgcct catccggcac actggagttt      360
aacaatgaaa gcttcaattg gactggagtc actcaaaatg gaacaagctc tgcttgcaaa      420
aggagatcta ataaaagttt ctttagtaga ttgaattggt tgacccactt aaaattcaaa      480
tacccagcat tgaacgtgac tatgccaaac aatgaaaaat ttgacaaatt gtacatttgg      540
ggggttcacc acccgggtac ggacaatgac caaatcagcc tatatgctca agcatcagga      600
agaatcacag tctctaccaa aagaagccaa caaactgtaa tcccgaatat cggatctaga      660
cccagggtaa gggatgtccc cagcagaata agcatctatt ggacaatagt aaaaccggga      720
gacatacttt tgattaacag cacagggaat ctaattgctc ctcggggtta cttcaaaata      780
cgaagtggga aaagctcaat aatgagatca gatgcaccca ttggcaaatg caattctgaa      840
tgcatcactc caaatggaag cattcccaat gacaaaccat ttcaaaatgt aaacaggatc      900
acatatgggg cctgtcccag atatgttaag caaaacactc tgaaattggc aacagggatg      960
cgaaatgtac cagagaaaca aactaga                                          987
```

<210> 8
<211> 1443
<212> DNA
<213> Artificial Sequence

<220>
<223> Synthetic Construct

<400> 8

```
atggcacaag tcattaatac aaacagcctg tcgctgttga cccagaataa cctgaacaaa      60
tcccagtccg ctctgggcac cgctatcgag cgtctgtctt ccggtctgcg tatcaacagc     120
gcgaaagacg atgcggcagg tcaggcgatt gctaaccgtt ttaccgcgaa catcaaaggt     180
ctgactcagg cttcccgtaa cgctaacgac ggtatctcca ttgcgcagac cactgaaggc     240
gcgctgaacg aaatcaacaa caacctgcag cgtgtgcgtg aactggcggt tcagtctgct     300
aacagcacca actcccagtc tgacctcgac tccatccagg ctgaaatcac ccagcgcctg     360
aacgaaatcg accgtgtatc cggccagact cagttcaacg gcgtgaaagt cctggcgcag     420
gacaacaccc tgaccatcca ggttggtgcc aacgacggtg aaactatcga tatcgatctg     480
aagcagatca actctcagac cctgggtctg gatacgctga atgtgcaaca aaaatataag     540
gtcagcgata cggctgcaac tgttacagga tatgccgata ctacgattgc tttagacaat     600
agtactttta aagcctcggc tactggtctt ggtggtactg accagaaaat tgatggcgat     660
ttaaaatttg atgatacgac tggaaaatat tacgccaaag ttaccgttac gggggggaact    720
ggtaaagatg gctattatga agtttccgtt gataagacga acggtgaggt gactcttgct     780
ggcggtgcga cttccccgct tacaggtgga ctacctgcga cagcaactga ggatgtgaaa     840
aatgtacaag ttgcaaatgc tgatttgaca gaggctaaag ccgcattgac agcagcaggt     900
gttaccggca cagcatctgt tgttaagatg tcttatactg ataataacgg taaaactatt     960
gatggtggtt tagcagttaa ggtaggcgat gattactatt ctgcaactca aaataaagat    1020
ggttccataa gtattaatac tacgaaatac actgcagatg acggtacatc caaaactgca    1080
ctaaacaaac tgggtggcgc agacggcaaa accgaagttg tttctattgg tggtaaaact    1140
tacgctgcaa gtaaagccga aggtcacaac tttaaagcac agcctgatct ggcggaagcg    1200
gctgctacaa ccaccgaaaa cccgctgcag aaaattgatg ctgctttggc acaggttgac    1260
acgttacgtt ctgacctggg tgcggtacag aaccgtttca actccgctat taccaacctg    1320
ggcaacaccg taaacaacct gacttctgcc cgtagccgta tcgaagattc cgactacgcg    1380
accgaagttt ccaacatggg cattttgcta ctattatcca tagctgtctt gattgctcta    1440
tcc                                                                  1443
```

<210> 9

<211> 481
<212> PRT
<213> Artificial Sequence

<220>
<223> Synthetic construct

<400> 9

Met Ala Gln Val Ile Asn Thr Asn Ser Leu Ser Leu Leu Thr Gln Asn
1               5                   10                  15

Asn Leu Asn Lys Ser Gln Ser Ala Leu Gly Thr Ala Ile Glu Arg Leu
            20                  25                  30

Ser Ser Gly Leu Arg Ile Asn Ser Ala Lys Asp Asp Ala Ala Gly Gln
        35                  40                  45

Ala Ile Ala Asn Arg Phe Thr Ala Asn Ile Lys Gly Leu Thr Gln Ala
    50                  55                  60

Ser Arg Asn Ala Asn Asp Gly Ile Ser Ile Ala Gln Thr Thr Glu Gly
65                  70                  75                  80

Ala Leu Asn Glu Ile Asn Asn Asn Leu Gln Arg Val Arg Glu Leu Ala
                85                  90                  95

Val Gln Ser Ala Asn Ser Thr Asn Ser Gln Ser Asp Leu Asp Ser Ile
            100                 105                 110

Gln Ala Glu Ile Thr Gln Arg Leu Asn Glu Ile Asp Arg Val Ser Gly
        115                 120                 125

Gln Thr Gln Phe Asn Gly Val Lys Val Leu Ala Gln Asp Asn Thr Leu
    130                 135                 140

Thr Ile Gln Val Gly Ala Asn Asp Gly Glu Thr Ile Asp Ile Asp Leu
145                 150                 155                 160

Lys Gln Ile Asn Ser Gln Thr Leu Gly Leu Asp Thr Leu Asn Val Gln
                165                 170                 175

Gln Lys Tyr Lys Val Ser Asp Thr Ala Ala Thr Val Thr Gly Tyr Ala
            180                 185                 190

Asp Thr Thr Ile Ala Leu Asp Asn Ser Thr Phe Lys Ala Ser Ala Thr
        195                 200                 205

Gly Leu Gly Gly Thr Asp Gln Lys Ile Asp Gly Asp Leu Lys Phe Asp
    210                 215                 220

```
Asp Thr Thr Gly Lys Tyr Tyr Ala Lys Val Thr Val Thr Gly Gly Thr
225                 230                 235                 240

Gly Lys Asp Gly Tyr Tyr Glu Val Ser Val Asp Lys Thr Asn Gly Glu
                245                 250                 255

Val Thr Leu Ala Gly Gly Ala Thr Ser Pro Leu Thr Gly Gly Leu Pro
            260                 265                 270

Ala Thr Ala Thr Glu Asp Val Lys Asn Val Gln Val Ala Asn Ala Asp
        275                 280                 285

Leu Thr Glu Ala Lys Ala Ala Leu Thr Ala Ala Gly Val Thr Gly Thr
    290                 295                 300

Ala Ser Val Val Lys Met Ser Tyr Thr Asp Asn Asn Gly Lys Thr Ile
305                 310                 315                 320

Asp Gly Gly Leu Ala Val Lys Val Gly Asp Asp Tyr Tyr Ser Ala Thr
                325                 330                 335

Gln Asn Lys Asp Gly Ser Ile Ser Ile Asn Thr Thr Lys Tyr Thr Ala
            340                 345                 350

Asp Asp Gly Thr Ser Lys Thr Ala Leu Asn Lys Leu Gly Gly Ala Asp
        355                 360                 365

Gly Lys Thr Glu Val Val Ser Ile Gly Gly Lys Thr Tyr Ala Ala Ser
    370                 375                 380

Lys Ala Glu Gly His Asn Phe Lys Ala Gln Pro Asp Leu Ala Glu Ala
385                 390                 395                 400

Ala Ala Thr Thr Thr Glu Asn Pro Leu Gln Lys Ile Asp Ala Ala Leu
                405                 410                 415

Ala Gln Val Asp Thr Leu Arg Ser Asp Leu Gly Ala Val Gln Asn Arg
            420                 425                 430

Phe Asn Ser Ala Ile Thr Asn Leu Gly Asn Thr Val Asn Asn Leu Thr
        435                 440                 445

Ser Ala Arg Ser Arg Ile Glu Asp Ser Asp Tyr Ala Thr Glu Val Ser
    450                 455                 460

Asn Met Gly Ile Leu Leu Leu Leu Ser Ile Ala Val Leu Ile Ala Leu
465                 470                 475                 480
```

Ser